Europäisches Patentamt

European Patent Office

Office européen des brevets

(11) Veröffentlichungsnummer: **0 296 279 B1**

## EUROPÄISCHE PATENTSCHRIFT

(45) Veröffentlichungstag der Patentschrift: **03.04.91**

(51) Int. Cl.5: **D04H 5/06, A61F 13/15**

(21) Anmeldenummer: **87115066.0**

(22) Anmeldetag: **15.10.87**

(54) Saugkörper aus Vliesstoff und Verfahren zu seiner Herstellung.

(30) Priorität: **16.06.87 DE 3720031**

(43) Veröffentlichungstag der Anmeldung:
**28.12.88 Patentblatt 88/52**

(45) Bekanntmachung des Hinweises auf die
Patenterteilung:
**03.04.91 Patentblatt 91/14**

(84) Benannte Vertragsstaaten:
**BE DE ES FR GB IT NL SE**

(56) Entgegenhaltungen:
**EP-A- 0 122 042**
**EP-A- 0 156 649**
**US-A- 3 414 444**
**US-A- 3 493 462**
**US-A- 4 333 979**

(73) Patentinhaber: **Firma Carl Freudenberg**
**Höhnerweg 2-4**
**W-6940 Weinheim/Bergstrasse(DE)**

(72) Erfinder: **Eschwey, Helmut, Dr.**
**Uhlandstrasse 25**
**W-6946 Gorxheimertal(DE)**
Erfinder: **Hackler, Lothar, Dr.**
**Höhenstrasse 32**
**W-6941 Abtsteinach(DE)**

Erfinder: **Hartmann, Ludwig, Dr.**
**Mozartstrasse 11**
**W-6940 Weinheim(DE)**
Erfinder: **Kauschke, Michael**
**In der Kappesdelle 31**
**W-6757 Waldfischbach-Burgalben(DE)**
Erfinder: **Klein, Bernhard, Dr.**
**Hofwiese 9**
**W-6943 Birkenau-Lo(DE)**
Erfinder: **Kümpel, Thomas**
**Albrecht-Dürer-Strasse 10**
**W-7518 Bretten(DE)**
Erfinder: **Kunkel, Hans-Achim**
**Panoramastrasse 2**
**W-6101 Reichelsheim(DE)**
Erfinder: **Nahe, Torsten**
**Unter den Weiden 5**
**W-6800 Mannheim 24(DE)**
Erfinder: **Ruzek, Ivo**
**Am Harzhübel 56**
**W-6750 Kaiserslautern(DE)**

## Beschreibung

Beschreibung

Die Erfindung befaßt sich mit einem Saugkörper aus Vliesstoff gemäß dem Oberbegriff des Anspruchs 1 und einem Verfahren zu seiner Herstellung. Seine Verwendungsmöglichkeiten liegen in medizinischen, hygienischen und techniscchen Bereichen, wo er Bestandteil von Flüssigkeiten aufnehmenden Produkten ist, bespielsweise von chirurgischen Saugtüchern, von Produkten zur Aufnahme von physiologishen Flüssigkeiten, wie Windeln, Inkontinenzeinlagen, Tampons und Damenbinden, oder von hochsaugfähigen Wischtüchern, sowie von Ölabscheidern.

Es ist bekannt, als hydrophiles saugfähiges Material eine Schicht aus zerfasertem, luftgelegtem Zellstoff, gegebenenfalls in Abmischung mit Synthesefasern, zu verwenden. Solche Saugkörper weisen erhebliche Mängel auf. Unter Belastung, beispielsweise unter dem Gewicht oder bei Bewegungen des Trägers einer Windel, wird die aufgenommene Flüssigkeit analog dem Auspressen eines Schwammes leicht wieder abgegeben. Ferner zerfasern kurze Zellstoff-Fasermatten bei Scherung und Knickung infolge ihres geringen Zusammenhaltes, wodurch die Kapillarkräfte und somit die Transportfähigkeit für die zu absorbierenden Flüssigkeiten aufgehoben werden. Wegen der Kürze der Cellulosefasern (1 bis 50 mm) und der lockeren Fluff-Legung herrscht eine ungenügende Kapillarfunktion vor, mit anderen Worten, der Transport von Flüssigkeit in den Faserzwischenräumen ist unvollkommen.

Somit wird weitaus weniger Flüssigkeit innerhalb des Saugkörpers weitergeleitet, als dieser theoretisch aufzunehmen in der Lage wäre.

Weiter ist es nachteilig, daß dessen Volumen im mit Flüssigkeit getränkten Zustand stark abnimmt: Der Fluff fällt in sich zusammen, seine Aufnahmekapazität verringert sich drastisch, Flüssigkeit tritt aus.

Die Erhöhung der Kapillarität und auch Naßfestigkeit eines Saugkörpers ist durch räumlich dichteres Anordnen der Zellstoff-Fasern möglich. Durch diese Maßnahme wird jedoch das der aufgenommenen Flüssigkeit zur Verfügung stehende Volumen drastisch verringert, wobei die eben genannte Verringerung der Aufnahmekapazität für Feuchtigkeit im getränkten Zustand noch hinzukommt. Wird als Ausweg die Fasermenge vervielfacht (z. B. mehrere Lagen aus Saugschichten), so entsteht ein allzu dickes und schweres Produkt, was eine erhebliche Erhöhung der Materialkosten zur Folge hat und die Handhabung sowie, z. B. bei Inkontinenzeinlagen, den Tragekomfort unzumutbar einschränkt.

Es hat nicht an Versuchen gefehlt, eine Lösung des Dilemmas zu finden, Saugkörpen einerseits eine hohe und dauerhafte Aufnahmekapazität für Flüssigkeiten zu verleihen (hohe Voluminosität, fast keine Kapillarfunktion) und andererseits über eine größere Faserdichte die Flüssigkeitsverteilung zwischen den Fasern erhöhen zu müssen (geringe Speicherkapazität).

Aus EP-A-156 649 ist ein gattungsgemäßer Saugkörper bekannt geworden, welcher Melt-Blown-Fasern als Matrix enthält, in welche kurze saugfähige Fasern und gegebenenfalls Quellkörper eingelagert sind. Dabei ist es jedoch erforderlich, eine genügende Verwicklung oder Verwirrung (entanglement) der Matrixfasern sicherzustellen. Diese besitzen nämlich keine ausreichende Festigkeit wegen ihrer herstellungsbedingt amorphen molekularen Struktur. Es herrscht somit in den Matrixfasern eine partielle Garnbildung vor, um deren genügende Festigkeit zu gewährleisten. Ferner ist es notwendig, daß diese Kurzfasern und das Quellpuder den Melt-Blown-Fasern direkt nach deren Herstellung, d.h. während diese noch klebrig aus der Düse kommen, zugemischt werden müssen. Der erforderliche, klebende Kontakt zwischen den Matrixfasern und den Zusätzen sätzen sowie die erwähnte mangelnde Festigkeit der Matrixfasern erfordern in jedem Falle einen Gewichtsanteil an letzteren von mindestens 50 %.

Die Aufgabe der vorliegenden Erfindung besteht darin, einen Saugkörper aus Vliesstoff anzugeben, der gleichermaßen eine gute Leitfähigkeit für Flüssigkeiten sowie eine gute Haltefähigkeit und Aufnahmekapazität aufweist. Er soll im Interesse des Tragekomforts (was im Falle von Inkontinenzartikeln wesentlich ist) eine geringere Dicke bei höherer Flexibilität und Stabilität aufweisen, als dies bei den gattungsgemäßen Saugkörper-Laminaten des Standes der Technik der Fall ist. Ferner sind für alle Einsatzgebiete, im hydrophilen oder gegebenenfalls im oleophilen Bereich, unabdingbar eine gute Festigkeit sowohl im trockenen wie auch im getränkten Zustand. Für den Fall, daß der Einbau von Superabsorber-Partikeln vorgesehen ist, sollen diese fest und gleichmäßig verteilt im Fasergefüge vorliegen und dennoch genügend Raum zum Quellen zur Verfügung haben. Der ungehinderte Transport von der Außenfläche des Saugkörpers hin zu den Quellkörpern muß gewährleistet sein. Dies alles soll mit möglichst wenig Matrixmaterial erreicht werden.

Ferner soll ein Verfahren aufgezeigt werden, das weniger Schritte als die bisherige Schichtenlegung und weniger Matrixfadenmaterial als bisherige Saugkörper erfordert.

Alle diese zum Teil bisher als widersprüchlich geltenden Anforderungen werden erfüllt durch einen

EP 0 296 279 B1

Saugkörper aus Vliesstoff, der hydrophile oder oleophile Fasern enthält, mit den kennzeichnenden Merkmalen des Anspruchs 1 und ein Herstellungsverfahren gemäß dem ersten Verfahrensanspruch. Jeweils vorteilhafte Ausgestaltungen werden in den Unteransprüchen aufgezeigt. Der erfindungsgemäße Saugkörper ist für alle genannten Gebiete in Medizin, Hygiene und Technik einsetzbar, wenn bei möglichst geringer Dicke ein hochsaugfähiges, naßfestes und eine Flüssigkeit haltendes Produkt erforderlich ist.

Es ist äußerst wesentlich für die vorliegende Erfindung, daß die Matrix aus endlosen Filamenten besteht, welche real ohne Unterbrechung sich von der einen bis zur anderen Seite des Saugkörpers erstrecken. Dabei besitzen diese Endlosfilamente einen hohen molekularen Orientierungsgrad, was ihre hervorragende Festigkeit bewirkt. Im Saugkörper sind sie wegen der noch zu schildernden Ablagetechnik räumlich willkürlich und kräuselartig verteilt. Ferner ist es sehr wesentlich für die Quellfähigkeit des Saugkörpers, daß sie gegenseitig im wesentlichen unverschlungen sind und einen kreisrunden Querschnitt besitzen.

Der Verlauf der Endlosfilamente im erfindungsgemäßen Saugkörper wird im folgenden als "dreidimensionale Streutextur" bezeichnet.

Es war überraschend, daß trotz dieses lockeren Aufbaus der Matrix sowohl die saugfähigen Kurzfasern in solch hohen Mengen wie 40 bis 90 Gew.-% wie auch gegebenenfalls bis zu 50 Gew.-% Quellkörper ohne Klebeverbindung sicher eingebunden werden. Als Vorteil resultiert hieraus selbstverständlich eine sehr hohe Feuchtigkeitsaufnahmeund Speicherfähigkeit, da die Matrixfasern aneinander gleitend leicht beweglich sind. Vorteilhaft ist ferner die Tatsache, daß schon bei einem Gehalt von nur 10% an Endlosfilamenten ausreichende Festigkeiten erhalten werden, was eine hohe Materialersparnis bedeutet.

Eine bezüglich ihrer Saugfähigkeit vorteilhafte Ausgestaltung ist durch einen zusätzlichen Gehalt von 2 bis 50 Gew.-% hochsaugfähiger, polymerer Quellkörper in räumlich gleichmäßiger Verteilung gekennzeichnet. Solche "Superabsorber" sind beispielsweise verseifte Stärke/Polyacryl-Pfropfpolymere, Stärke/Polyacrylatpfropfpolymere, vernetzte oder gepfropfte Cellulose, verseifte Acrylsäurepolymere und verseifte Acrylsäurecopolymere, vernetzte Polyethylenoxide oder Carboxymethylcellulosen. Bewährt haben sich saugfähige Flächengebilde, die teilvernetzte Polyacrylsäuredevirate oder Stärke/Pfropfcopolymere enthalten.

Die Endlosfilamente bilden mit ihrer räumlichen Streutextur eine selbsttragende, in Längs- und Querrichtung naß- und trockenfesten Matrix für alle hydrophilen oder oleophilen Komponenten.

Falls eine besonders hohe Flüssigkeitsverteilung gewünscht wird, wird als vorteilhafte Variante ein stark verdichteter Saugkörper vorgeschlagen, bei dem die Quellkörper an den sie umgebenden Fasern und Filamenten haften. Die thermoplastischen Endlosfilamente der Matrix sind jedoch nur an deren Oberfläche leicht miteinander schmelzverbunden; die freie Beweglichkeit der Filamente im Inneren der Matrix darf nicht durch Schmelzklebepunkte behindert sein und begünstigt weiterhin die ungehinderte Quellung des Superabsorbers. Somit kann die Aufnahmefähigkeit für Flüssigkeiten bei Anwendung der Erfindung auch in komprimierten Produkten sehr hoch eingestellt werden. Die Quellkörper erhöhen zudem das Haltevermögen für Flüssigkeiten.

Dicke und Dichte des erfindungsgemäßen Saugkörpers können innerhalb weiter Grenzen den jeweiligen Erfordernissen angepaßt werden, ohne den Rahmen der vorliegenden Erfindung zu verlassen.

Überraschend ist das gute Flüssigkeitsverteilungsvermögen: Nach dem Stand der Technik erwartet man, daß ein homogenes Gemisch aus Superabsorber und hydropilen Fasern zu starker Gelblockbildung neigt, die Flüssigkeitsverteilung also stärker behindert wird als in einem lose gelegten Fluff. Überraschenderweise beeinträchtigt jedoch die erfindungsgemäße Ausführungsform des Saugkörpers die Flüssigkeitsverteilung in keiner Weise, was mit der Streutextur-Lage der Endlosfilamente als Gerüst erklärt werden kann.

Die hydrophilen Fasern, welche primär die Nässeaufnahme bewirken, bestehen aus den an sich bekannten Materialien Zellstoff, Zellwolle, Baumwolle, Linters oder Holzschliff.

Ein bevorzugtes Anwendungsgebiet des erfindungsgemäßen Saugkörpers sind Inkontinenzartikel für Kleinkinder und Erwachsene, wie Wegwerfwindeln, Windelhosen, Damenbinden und Betteinlagen. Diese Artikel bestehen im wesentlichen aus einer unteren, flüssigkeitsundurchlässigen Schicht (meistens Folie), einer oberen, hydrophoben, flüssigkeitsdurchlässigen Schicht (Faservlies oder perforierte Folie) und dem erfindungsgemäßen Saugkörper zwischen diesen genannten Schichten.

Allen solchermaßen beschaffenen Inkontinenzartikeln gemeinsam ist die erhebliche Verbesserung des Tragekomforts:

Die nasse Saugschicht hält auch dann zusammen, wenn sich der Träger bewegt. Das Fasergefüge des Flüssigkeitsspeichers wird dabei nicht unterbrochen; Flüssigkeit tritt nicht aus.

Verklumpungen des nassen Superabsorbers, wie sie insbesondere in Zellstoff-Fasermatten bei Erwachsenenwindeln häufig störend auftreten, werden vermieden. Die bereits ohne weitere Verfestigung guten

3

EP 0 296 279 B1

Festigkeitswerte in Längs- und Querrichtung machen den Saugkörper auch für den Einsatz in Tampons sowie in medizinischen Tupfern vorteilhaft.

Im technischen Bereich ist der erfindungsgemäße Saugkörper in seiner hydrophilen Variante als Superabsorber enthaltendes Quellvlies für Kabelummantelungen einsetzbar: Mittel-und Hochspannungskabel enthalten diese Quellbandagen zwischen Mantel und Abschirmung, um im Falle einer Beschädigung des Mantels eintretendes Wasser sofort binden und fixieren und gleichzeitig die Schadstelle durch sofortiges, intensives Quellen dichtend verschließen zu können. Die Anforderungen sind also die gleichen wie bei den Hygieneprodukten: Möglichst geringe Dicke bei möglichst hoher Quellfähigkeit und ungehinderter Ausdehnung der Quellpartikeln.

Die herausragenden Festigkeiten des erfindungsgemäßen Saugkörpers favorisieren ihn ebenso als alleinigen Bestandteil oder als saugfähige Komponente eines Wischtuches. Wieder kommen dabei die günstigen Eigenschaften des erfindungsgemäßen Saugkörpers, wie geringe Dicke und dennoch hohe Saugfähigkeit, hohes Flüssigkeitshaltevermögen und hohe innere Eigenfestigkeit, auch im getränkten Zustand, zum Tragen.

Durch die Verwendung von oleophilen Fasern besteht die Möglichkeit, den erfindungsgemäßen Saugkörper als Depot oder Absorber für ölhaltige Substanzen einzusetzen. Darunter versteht die Anmelderin Stoffe, die eine Affinität zu oleophilen Materialien besitzen. Damit sind die Abtrennung von Ölen aus Wasser und Luft sowie die Verwendung des erfindungsgemäßen Gegenstandes im medizinischen oder kosmetischen Bereich denkbar.

Solche oleophilen Fasern sind z. B. Stapelfasern aus Polyolefinen. Als besonders vorteilhaft bei der Abtrennung von Öl aus Wasser hat sich in neuester Zeit der Einsatz von olephiliertem Holzschliff erwiesen.

Als zu Endlosfilamenten verspinnbare Substanzen eignen sich Polypropylen, Polyethylen, Polyester, Polyamid oder Polyurethan.

Die Endlosfilamente werden aus der Schmelze mittels einer oder mehrerer Düsen ersponnen. Dabei werden sie durch einen nach unten gerichteten Luftstrom verstreckt und somit ihr molekularer Aufbau stark orientiert. Erst im unteren Drittel der Weglänge zwischen Spinndüse und Ablageband werden die hydrophilen oder oleophilen Fasern und gegebenenfalls die Superabsorber-Partikeln zugegeben. In diesem Bereich dürfen die Filamente auf keinen Fall mehr klebefähig sein. Die Bewegung der Unterlage wird so gesteuert, daß sie sich wesentlich langsamer bewegt, als es der Spinngeschwindigkeit entspricht. Dabei entsteht die kräuselartige Ablageform der Endlosfilamente mit ihrer "räumlichen Streutextur" und somit eine ThermoplastMatrix, in welcher die hydrophilen Fasern, gegebenenfalls mit Superabsorber, eingeschlossen sind. Der so hergestellte Saugkörper ist ohne weitere Verfestigung für den normalen, insbesondere einmaligen, Gebrauch verwendbar; das Auspulvern von Fasern oder Partikeln ist vernachlässigbar gering.

Für besonders beanspruchte Wischtücher kann es vorteilhaft sein, das Faserflächengebilde zusätzlich unter Druck- und Wärmeeinwirkung zu verdichten und zu verfestigen. Die angewendete Wärme muß dabei ausreichen, die Endlosfilamente an der Matrixoberfläche klebefähig zu machen. Sie muß jedoch so dosiert werden, daß die freie gegenseitige Beweglichkeit der Endlosfilamente im Inneren der Matrix gewährleistet bleibt. Dieses Maß ist durch Vorversuche für den jeweiligen Gehalt an Endlosfilamenten zu ermitteln; je nach dem gewünschten Verdichtungsgrad erfolgt eine vollflächige, eine Linien- oder eine Punktprägung, wobei die Verfestigung in jedem Fall nur die Oberfläche des Saugkörpers betreffen darf.

Als besonders vorteilhaft wurde bei Anwesenheit von Superabsorber-Partikeln, die in Mengen von 2 bis 50 Gew.-%, bezogen auf das Gesamtgewicht, zugegeben werden können, die vollflächige Verdichtung des Flächengebildes in feuchter Atmosphäre bei einem Liniendruck bis maximal 150 kp/cm gefunden. Die Quellkörper haften unter der Einwirkung der Feuchtigkeit aneinander und an den sie umgebenden Fasermaterialien, ohne jedoch aufzuquellen. Somit wird eine Verfestigung der Strukturen erzielt, welche sich von selbst bei Zutritt von Flüssigkeit wieder löst und das ungehinderte Quellen ermöglicht.

Es muß nochmals betont werden, daß der Grad des Verpressens je nach dem Einsatzbereich des erfindungsgemäßen Saugkörpers stark variiert werden kann, ohne die Grundidee der vorliegenden Erfindung zu verlassen. Werden aus dem Saugkörper z. B. Wischtücher hergestellt, so wird eine stärkere Verfestigung der Oberfläche durchgeführt werden müssen, um eine höhere Abriebfestigkeit zu erreichen. Dies kann sowohl durch Kalanderverfestigung mit einer Prägewalze als auch zweckmäßig durch Aufsprühen eines Bindemittels erfolgen.

Sollen aus dem saugfähigen Flächengebilde Windeln hergestellt werden, wird man im allgemeinen ein größeres Volumen anstreben und die Oberflächenverfestigung nicht so weit treiben, da bei der Windelherstellung im allgemeinen eine Abdeckung die Saugschicht vor Abrieb schützt.

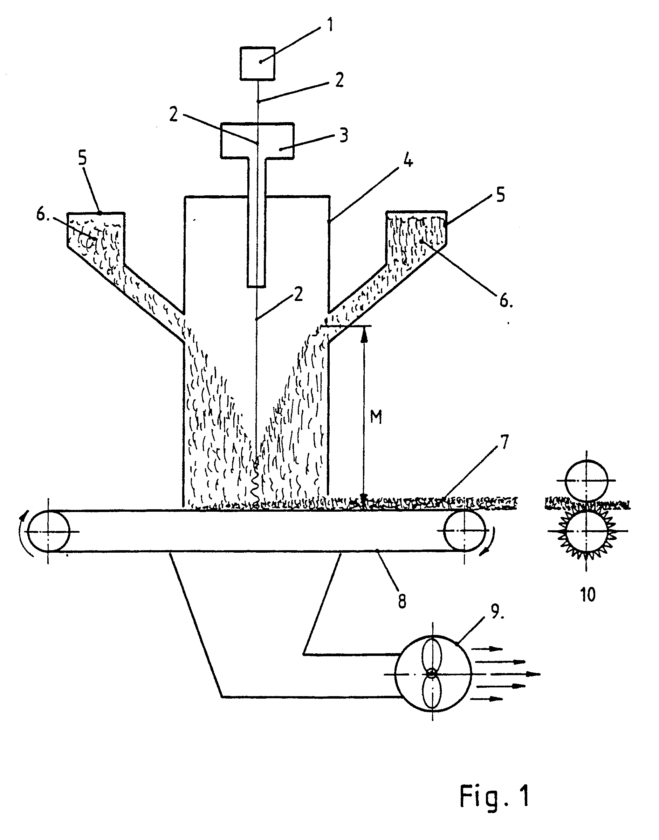

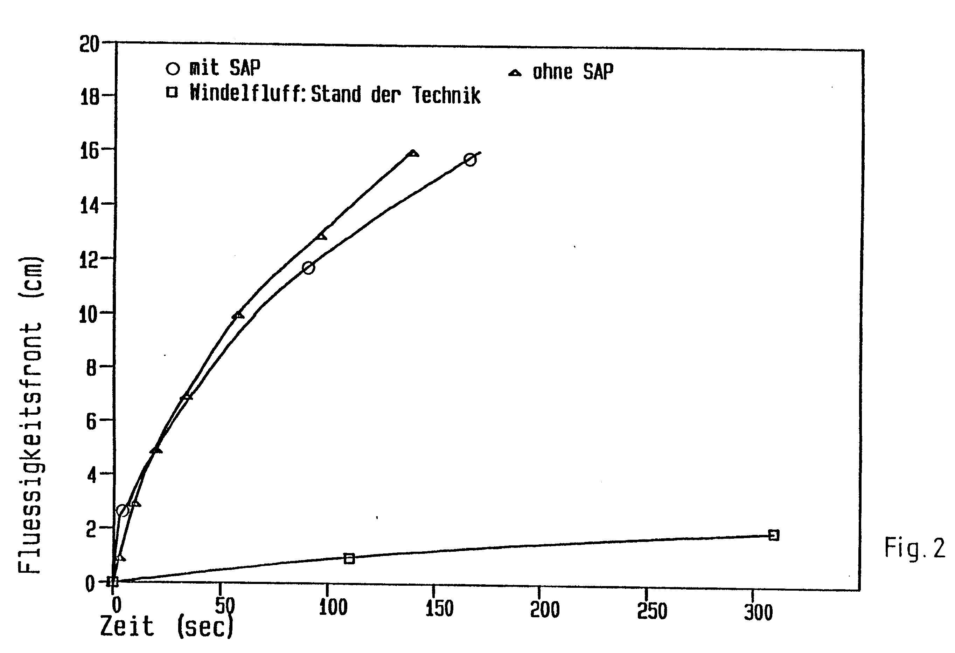

Figur 1 zeigt in halbschematischer Darstellung beispielhaft eine Vorrichtung zur Durchführung des erfindungsgemäßen Verfahrens, Figur 2 den Vergleich zwischen einem Windelfluff und zwei erfindungsgemäßen Saugkörpern bezüglich der Flüssigkeits-Leitfähigkeit.

4

Aus Figur 1 wird der prinzipelle Ablauf des Herstellungsverfahrens deutlich, bei dem über eine Längsspinndüse 1, die eine oder mehrere Reihen von Spinnöffnungen besitzt, eine entsprechende Anzahl von endlosfilamentreihen 2 ersponnen wird. Diese durchlaufen einen Führungs- und Verstreckkanal 3, wo sie mit parallel zur Fallrichtung geblasener Luft in einem gewünschten Ausmaß verstreckt werden. Der Führungskanal und damit die Endlosfilamente münden sodann in einen Schacht 4, dessen unteres Drittel den Mischungsbereich M mit den aus einem Mahlwerk 5 zugegebenen, hydrophilen oder oleophilen Fasern 6 sowie gegebenenfalls den an gleicher Stelle zugegebenen Quellkörper-Partikeln bildet. Luftströme können dabei die Zugabe der Fasern und Partikeln fördern; diese Zugabe erfolgt beidseitig der Filamentreihen.

Die Filamente und Fasern mit gegebenenfalls den Quellkörpern treffen auf ein sich gleichförmig bewegendes Endlos-Siebband 8, auf dessen Oberfläche sie mittels einer Saugvorrichtung 9 festgehalten werden. Die abgesaugte Luft kann nach Passieren einer Reinigungsvorrichtung wieder dem Verstreckungsbereich der Anlage zugeführt werden.

Das entstehende Flächengebilde 7 gelangt sodann zur Komprimierung und Verfestigung in ein beheiztes Prägewerk 10 mit einer glatten und einer Gravurwalze.

Im folgenden werden beispielhaft zwei Ausführungsformen des erfindungsgemäßen Saugkörpers beschrieben, deren eine mit Superabsorber (im folgenden SAP genannt) versehen ist. Beiden Versionen wird ein Windelfluff gemäß dem Stand der Technik gegenübergestellt, wie er als saugfähige Komponente in Babywindeln eingesetzt wird.

| | Erfindungsgemäßer Saugkörper | | Windelsaugschicht |
|---|---|---|---|
| | Beispiel 1 | Beispiel 2 | (Stand d.Technik) |
| Aufbau | 10 Gew.-% Endlosfilamente (Polypropylen), Restfasern Fluffzellstoff | | 100 Gew.-% Fluffzellstoff |
| Superabsorber (SAP) | 25 Gew.-% | – | – |
| Flächengewicht $(g/m^2)$ | 300 | 300 | 500 |
| Dicke (mm) | 1,25 | 1,25 | 6,05 |
| Dichte $(g/cm^3)$ | 0,24 | 0,24 | 0,083 |
| Höchstzugkraft (N/ 5 cm) | | | |
| trocken/längs | 20,6 | 38,9 | ) |
| trocken/quer | 29,4 | 38,6 | ) nicht meßbar |
| naß/längs | 6,4 | 14,9 | ) |
| naß/quer | 10,6 | 9,8 | ) |
| Höchstzugkraft- dehnung (%) | | | |
| trocken/längs | 35 | 80 | ) nicht meßbar |
| trocken/quer | 34 | 73 | ) |
| Flüssigkeits- aufnahme- Kapazität (g/g) | | | |
| $TBA_{30}$ | 11,4 | 7,9 | 17,2 |
| $TBA_{600}$ | 23,5 | 9,3 | 19,1 |
| $TBR_{30}$ | 7,4 | 2,3 | 2,8 |
| $TBR_{600}$ | 8,7 | 3,5 | 3,1 |

TBA/TBR: Absorption/Retention im "Teebeuteltest"

Indizes: Sekunden

Zur Bestimmung der Absorption/Retention nach dem Teebeuteltest wird so vorgegangen, daß eine genau eingewogene Probenmenge in einen Teebeutel eingeschweißt und für gegebene Zeitintervalle in die Testflüssigkeit eingetaucht wird, um sich vollzusaugen. Man läßt 5 Minuten abtropfen, wiegt aus, zentrifugiert anschließend in einer handelsülichen Wäscheschleuder bei 1.400 U/min und wiegt erneut. Die Absorption (A) wird errechnet:

$$A = \frac{\text{Masse (Teebeutel mit Probe nach dem Abtropfen – Referenzprobe)}}{\text{Trockenmasse der Probe}}$$

Die Retention (R) wird errechnet:

$$R = \frac{\text{Masse (Teebeutel mit Probe nach dem Schleudern - Referenzprobe)}}{\text{Trockenmasse der Probe}}$$

Die Referenz wird mit einem leeren Teebeutel unter gleichen Bedingungen ermittelt.

Mit dem Spreittest wird die Sauggeschwindigkeit einer Flüssigkeit in der Probe bestimmt (siehe Figure 2). Dabei läßt sich die Neigung der Saugschicht zwischen 0° und 60° einstellen.

Die Vorrichtung hierzu besteht aus einer Wanne (8 cm x 25 cm), an deren Stirnseite eine Vertiefung eingearbeitet ist. In dieser taucht die Probe während der Messung in die Testflüssigkeit ein.

Die Messung der Sauggeschwindigkeit erfolgt automatisch über eine Leitfähigkeitsmessung. Während eine Elektrode in die Testflüssigkeit taucht, werden die Gegenelektroden in die Saugschicht gesteckt.

Wenn die Front der elektrisch leitfähigen Flüssigkeit die erste Nadel erreicht, fließt ein definierter Strom. Mit fortschreitender Flüssigkeitsfront erhöht sich der Strom beim Erreichen jeder weiteren Nadel um den gleichen Betrag. Mit einem Schreiber wird der Meßstrom in Abhängigkeit von der Zeit festgehalten.

Testflüssigkeit Syntheseurin

19,4 g Harnstoff
8,3 g NaCl
1,0 g $MgSO_4$
0,6 g $CaCl_2$
970,7 g destilliertes Wasser

Der Saugkörper aus Beispiel 1 der vorstehenden Tabelle enthält Superabsorber und verteilt als sehr dünnes Produkt die Flüssigkeit sehr gut. Aus Figur 2 wird deutlich, wie seine Saugschicht nach kurzer Zeit aufquillt und sogar die Kapazität von herkömmlichem Windelzellstoff (TBA $_{600}$) übersteigt. Auch das Rückhaltevermögen (TBR$_{600}$) ist mit 8,7 g/g wesentlich besser als im Falle der Babywindel. Sehr gut sind auch die Werte für die Naß- und Trockenfestigkeit (Tabelle), die man mit Saugschichten herkömmlicher Windeln nicht bestimmen kann, weil diese keinerlei Eigenfestigkeit haben.

Der Saugkörper nach Beispiel 2 enthält keinen Superabsorber und hat deshalb fast kein Rückhaltevermögen. Bezüglich Festigkeit und Flüssigkeits-Verteilungsvermögen ist er jedoch ebenfalls herkömmlichem Windelfluff weit überlegen, wie aus der Tabelle und Figur 2 hervorgeht.

**Ansprüche**

1. Saugkörper aus einem ein einschichtiges, sich durch-dringendes Netzwerk darstellenden Vliesstoff mit einer Gesamtdichte von 0,05 bis 0,5 g/cm³, bestehend aus einer Matrix von thermoplastischen Filamenten und enthaltend räumlich gleichmäßig verteilt hydrophile oder oleophile, 1 bis 50 mm lange Fasern, dadurch gekennzeichnet, daß die hydrophilen oder oleophilen Fasern in einer Menge von 40 bis 90 Gew.-% enthalten sind und daß die Matrix gebildet wird aus Endlosfilamenten mit einem hohen molekularen Orientierungsgrad, die räumlich willkürlich und kräuselartig im Vliesstoff vorliegen und einen kreisrunden Querschnitt besitzen.

2. Saugkörper nach Anspruch 1, dadurch gekennzeichnet, daß er zusätzlich 2 bis 50 Gew.-% polymere Quellkörper in räumlich gleichmäßiger Verteilung eingelagert enthält.

3. Saugkörper nach Anspruch 2, dadurch gekennzeichnet, daß er an seiner Oberfläche stark verdichtet ist, wobei die Endlosfilamente im Inneren der Matrix nicht miteinander schmelzverbunden sind, und daß die Quellkörper an den sie umgebenden Fasern und Filamenten haften.

4. Saugkörper nach Anspruch 1 bis 3, dadurch gekennzeichnet, daß die hydrophilen Fasern aus Zellstoff, Zellwolle, Baumwolle, Linters oder Holzschliff bestehen.

5. Saugkörper nach Anspruch 1 bis 3, dadurch gekennzeichnet, daß die oleophilen Fasern aus Polyolefinen oder oleophiliertem Holzschliff bestehen.

6. Verfahren zur Herstellung eines Saugkörpers nach den Ansprüchen 1 bis 5, wobei man die Matrixfilamente aus der Schmelze mittels einer oder mehrerer Düsen erspinnt und zusammen mit hydrophilen oder oleophilen Fasern von 1 bis 50 mm Länge auf eine gleichförmig horizontal bewegte, luftdurchlässige und mit einer unterhalb befindlichen Luft-Absaugvorrichtung versehene Unterlage auftreffen läßt und wobei man die Matrixfilamente durch einen Luftstrom nach unten verstreckt und dabei Kurzfasern zugibt, dadurch gekennzeichnet, daß man die Filamente endlos verspinnt und daß man die Kurzfasern etwa im unteren Drittel zwischen Spinndüse und Ablageband in einer Menge von 40 bis 90 Gew.-%, bezogen auf das gesamte Fasergewicht, dem verstreckenden Luftstrom zugibt, wobei die Fasern erst mit den nicht mehr klebefähigen Endlosfilamenten in Berührung kommen, und daß man die Unterlage lage wesentlich langsamer bewegt, als es der Spinngeschwindigkeit der Endlosfilamente entspricht, so daß diese räumlich willkürlich und kräuselartig abgelegt und mit den hydrophilen oder oleophilen Fasern gleichmäßig vermischt werden.

7. Verfahren nach Anspruch 6, dadurch gekennzeichnet, daß man in gleicher Weise, an gleicher Stelle wie die und gleichzeitig mit den kurzen Fasern polymeres Quellkörpermaterial in Mengen von 2 bis 50 Gew.-%, bezogen auf das Gesamtgewicht des abgelegten Materials, zugibt.

8. Verfahren nach Anspruch 7, dadurch gekennzeichnet, daß man nach der Ablage das Flächengebilde mit einem Liniendruck von max. 150 kp/cm verdichtet unter Einwirkung einer Feuchtigkeitsmenge, die so bemessen ist, daß sie die Quellkörper-Oberflächen klebrig werden läßt, ohne jedoch den Quellvorgang einzuleiten.

## Claims

1. An absorbent article formed from a nonwoven representing a single-layered, interpenetrating network, having a total density of from 0.05 to 0.5 g/cm$^3$, comprising ing a matrix of thermoplastic filaments and containing hydrophilic or oleophilic fibres from 1 to 50 mm in length in a spatially uniform distribution, characterized in that the hydrophilic or oleophilic fibres are present in an amount of from 40 to 90% by weight and in that the matrix is formed from continuous filaments which possess a high degree of molecular orientation, which are spatially randomly and curlily present in the nonwoven and which have a circular cross-section.

2. An absorbent article according to claim 1, characterized in that it additionally contains from 2 to 50% by weight of polymeric superabsorber particles embedded in a spatially uniform distribution.

3. An absorbent article according to claim 2, characterized in that it has been strongly compacted at its surface, although the continuous filaments in the interior of the matrix have not been fuse-bonded to one another, and in that the superabsorber particles adhere to the surrounding fibres and filaments.

4. An absorbent article according to claims 1 to 3, characterized in that the hydrophilic fibres comprise cellulose, staple viscose, cotton, linters or groundwood.

5. An absorbent article according to claims 1 to 3, characterized in that the oleophilic fibres comprise polyolefins or oleophilicized groundwood.

6. A process for producing an absorbent article according to claims 1 to 5, wherein the matrix filaments are spun from the melt by means of one or more dies and are allowed to impinge together with hydrophilic or oleophilic fibres from 1 to 50 mm in length on an airpermeable surface which is moving horizontally at a uniform speed and has been provided underneath with airaspirating means and wherein the matrix filaments are oriented in a downward direction by an airstream while short fibres are added, characterized in that the filaments ments are spun as continuous filaments and in that the short fibres are added to the orienting airstream in approximately the bottom third between the spinning die and the collecting belt in an amount of from 40 to 90% by weight, based on the total fibre weight, the fibres only coming into contact with the continuous filaments when the latter are no longer tacky, and in that the surface moves at a significantly lower speed than corresponds to the spin speed of the continuous filaments, so that they come to be laid spatially randomly and curlily and are uniformly mixed with the hydrophilic or oleophilic fibres.

7. A process according to claim 6, characterized in that polymeric superabsorber material is added in the same way, in the same area and at the same time as the short fibres in amounts of from 2 to 50% by weight, based on the total weight of the deposited material.

8. A process according to claim 7, characterized in that, after deposition, the sheetlike structure is compacted with a linear pressure of at most 150 kp/cm in the presence of an amount of moisture sufficient to make the surfaces of the superabsorber particles tacky without. however, initiating the process of swelling.

**Revendications**

1. Corps absorbant constitué d'une nappe de fibres à une couche formant un réseau entreinêlé avec une densité globale de 0,05 à 0,5 g/cm³, constitué d'une matrice de filaments thermoplastiques et contenant des fibres hydrophiles ou oléophiles de 1 à 50 mm de longueur réparties uniformément dans l'espace, caractérisé en ce que les fibres hydrophiles ou oléophiles sont présentes à raison de 40 à 90% en poids et que la matrice est constituée de filaments sans fin avec un degré d'orientation moléculaire élevé qui sont présents dans la nappe de fibres avec répartition au hasard dans l'espace et avec des ondulations et qu'ils possèdent une section circulaire.

2. Corps absorbant selon la revendication 1, caractérisé en ce qu'il contient en outre de 2 à 50% en poids de corps gcnflant polymère réparti uniformément dans l'espace.

3. Corps absorbant selon la revendication 2, caractérisé en ce qu'il est fortement compacté à sa surface tandis que les filaments sans fin ne sont pas assemblés les uns aux autres par fusion à l'intérieur de la matrice et que les corps absorbants adhèrent aux fibres et filaments environnants.

4. Corps absorbant selon les revendications 1 à 3, caractérisé en ce que les fibres hydrophiles sont constituées de cellulose, de laine de cellulose, de coton, de linters ou de pâte de bois mécanique.

5. Corps absorbant selon les revendications 1 à 3, caractérisé en ce que les fibres oléophiles sont constituées de polyoléfines ou de pâte de bois mécanique oléophilisée.

6. Procédé de fabrication d'un corps absorbant selon les revendications 1 à 5, dans lequel les filaments de la matrice sont filés hors du bain fondu au moyen d'une ou de plusieurs tuyères et sont amenés avec les fibres hdyrophiles ou oléophiles d'une longueur de 1 à 50 mm sur une couche de base perméable à l'air et se déplaçant d'un mouvement horizontal, pourvue d'un dispositif d'aspiration de l'air disposé en dessous de celle-ci et dans lequel les filaments de la matrice sont dressés vers le bas par un courant d'air avec addition de fibres courtes, caractérisé en ce que les filaments sont filés sans fin et que les fibres courtes sont ajoutées à peu près dans le troisième tiers entre la tuyère de filage et la bande de dépôt en proportion de 40 à 90% en poids rapportée au poids total des fibres en étant introduites dans le courant d'air de dressage tandis que les fibres viennent en contact avec les filaments sans fin qui ne sont plus adhérents et que la couche de base se déplace essentiellement plus lentement que ce qui correspondrait à la vitesse de filage des filaments sans fin, si bien que ceux-ci sont déposés au hasard dans l'espace et avec des ondulations et sont mélangées uniformément aux fibres hydrophiles ou oléophiles.

7. Procédé selon la revendication 6, caractérisé en ce que l'on ajoute un matériau polymère gonflant en quantité de 2 à 50% en poids rapportée au poids total des matériaux déposés de la même manière, au même endroit et au même moment que les fibres courtes.

8. Procédé selon la revendication 7. caractérisé en ce que, après le dépôt des matériaux, l'élément plat est compacté avec une pression linéaire de 150 kp/cm au maximum en faisant agir une quantité d'humidité choisie de façon à ce que la surface des corps gonflants devienne collante sans toutefois provoquer le processus de gonflement.

Fig. 1

Fig. 2